# EUROPEAN PATENT APPLICATION

(11) **EP 2 177 612 A1**
(43) Date of publication of application: **21.04.2010**
(21) Application number: 08791131.9
(22) Date of filing: 14.07.2008
(51) Int. Cl.: C12N 15/09, C12Q 1/68, G01N 33/53, G01N 33/542, G01N 33/566

(54) **METHOD FOR OBTAINING INFORMATION ON FORMATION OF DOUBLE-STRANDED NUCLEIC ACID**

(30) Priority: 14.08.2007 JP 2007211366
(71) Applicant: Sony Corporation, Tokyo 108-0075 (JP)
(72) Inventor: ABE, Tomoteru, Tokyo 108-0075 (JP)
(74) Representative: Sexton, Jane Helen
(86) International application number: PCT/JP2008/062706
(87) International publication number: WO 2009/022512

(57) **Abstract**

Provided is a method for obtaining information on the formation of a double-stranded nucleic acid by detecting fluorescence, which is simple, is applicable to various interaction systems, and features high detection sensitivity with reduced background signals. Specifically provided is a method for obtaining information on the formation of a double-stranded nucleic acid, which includes detecting information on fluorescence of a probe consisted of a labeling fluorescent dye labeled on a nucleic acid strand and an intercalator bound or inserted between base pairs in the double-stranded nucleic acid to permit an energy transfer with the fluorescent dye.

## Description

### Technical Field

This invention relates to a method for obtaining information on the formation of a double-stranded nucleic acid. More specifically, the present invention is concerned with a method for obtaining information on the formation of a double-stranded nucleic acid simply and with high sensitivity.

### Background Art

Technologies, each of which detects hybridization, a double-stranded nucleic acid or the like by measuring fluorescence originated from a fluorescent dye labeled on a nucleic acid, a fluorescent dye bound or inserted between base pairs in the double-stranded nucleic acid for the emission of fluorescence, or the like (hereinafter referred to as "fluorescence detection technology or technologies"), have already found practical utility in various fields of molecular biology such as Southern blotting, Northern blotting, DNA microarray and real-time PCR.

The above-described fluorescence detection technologies include a method that firstly adds a fluorescently-labeled target nucleic acid strand to a region of interaction, where a probe nucleic acid strand is immobilized, to conduct hybridization, washes the region of interaction with a predetermined solution, and then detects fluorescence originated from the target nucleic acid strand. By this method, it is possible to determine whether or not a nucleic acid having a base sequence complementary to that of the probe nucleic acid strand exists in the target nucleic acid strand, namely, to obtain information on the expression of a specific gene, information on the amount of a genomic DNA of a germ, or the like.

This method is used in Southern blotting, Northern blotting, DNA microarray, and the like.

Also used as a second technology is a technology that detects hybridization, for example, by using a fluorescent dye called "intercalator" which inserts (intercalates) between complementary base pairs in a double-stranded nucleic acid and emits fluorescence. This detection technology has an advantage that it does not require a dedicated probe for every gene and can reduce experiment cost.

This method is used in real-time PCR and the like.

In recent years, a method that detects amplification of a target gene by using a specific fluorescent probe has been put to practical use. For example, in accordance with a method that uses a probe nucleic acid strand modified at a 5'-end thereof with a fluorescent dye and at a 3'-end thereof with a quencher substance capable of quenching fluorescence of the fluorescent dye ("TaqMan^{™}" probe), amplification of a target gene can be detected by measuring fluorescence emitted from the fluorescent dye located apart from the quencher as a result of a progress of a polymerase reaction.

Japanese Patent Laid-open No. Hei 11-290098 discloses a detection method of a double-stranded nucleic acid, which consists measuring a change in a fluorescence characteristic caused by an interaction of a 9-azoacridine derivative with the double-stranded nucleic acid.

Disclosed in Japanese Patent Laid-open No. 2005-291703 is a fluorescent reagent of an (α-amino acid residual group)-containing peptide, which consists two or more α-amino acid residual groups substituted with fluorescent intercalation groups, respectively. Specifically disclosed is a fluorescent reagent for the detection of a double-stranded nucleic acid containing fluorescent intercalation groups. Upon contact with the double-stranded nucleic acid, the fluorescent intercalation groups in fluorescent reagent molecules intercalate between base pairs in the double-stranded nucleic acid, so that quenching due to the stacking of fluorescent intercalation groups in the fluorescent reagent molecules is cancelled to lead to an increase in fluorescence intensity. This increase makes it possible to detect the double-stranded nucleic acid.

Japanese Patent Laid-open No. 2001-245699 discloses a detection method of a nucleic acid, which is characterized by including a step of conducting formation of a hybrid between a probe, which is consisted of a single-stranded polynucleotide labeled with an energy donor and an energy acceptor, and a labeled nucleic acid, and another step of detecting a change in fluorescent emission from the energy donor or energy acceptor by irradiation of a laser between before and after the formation of the hybrid, and detecting the target nucleic acid by detecting based on the change in fluorescent emission whether or not the formation of the hybrid has taken place or not.

However, the above-described fluorescence detection technologies are accompanied by problems in that a limitation is imposed on applicable interaction systems and no sufficient sensitivity is obtainable.

Described specifically, the first technology requires to wash and eliminate the fluorescently-labeled target nucleic acid strand after the hybridization. Therefore, the probe nucleic acid strand needs to be immobilized at a predetermined region of interaction, thereby making it impossible to use the first technology in hybridization or the like in a solution.

The second technology does not require the washing and elimination of the intercalator, and therefore, can also be used for the detection of hybridization or the like in a solution. The second technology is, however, accompanied by a shortcoming in that, due to detection of fluorescence of the intercalator liberated into the solution, the intercalator inserted between base pairs in a byproduced, double-stranded nucleic acid, and the like, background signals are large and the sensitivity is not necessarily high.

The third technology requires to design two types of probes specific to a detection-target such that these probes hybridize side by side. The third technology is, therefore, accompanied by a shortcoming that the designing of these probes is difficult to result in high cost.

The present invention, therefore, has as a primary object thereof the provision of a method for obtaining information on the formation of a double-stranded nucleic acid by detecting fluorescence, which is simple, is applicable to various interaction systems, and features high detection sensitivity with reduced background signals.

### Disclosure of Invention

To solve the above-described technical problem, the present invention firstly provides a method for obtaining information on formation of a double-stranded nucleic acid between a first nucleic acid strand and a second nucleic acid strand having a base sequence complementary to that of the first nucleic acid strand, which includes detecting information on fluorescence of a probe consisted of a labeling fluorescent dye labeled on the first nucleic acid strand and an intercalator bound or inserted between base pairs in the double-stranded nucleic acid to permit an energy transfer with the fluorescent dye. According to this method, background signals can be reduced upon detection of fluorescence by making use of a fluorescence resonance energy transfer (FRET) between probe molecules.

The probe in the method according to the present invention can be included of a group of plural fluorescent dyes including at least an excitation-target fluorescent dye capable of giving excitation energy, which has been obtained by absorption of light, to another fluorescent dye and a detection-target fluorescent dye capable of fluorescing by receiving the excitation energy from the another fluorescent dye.

The intercalator in the present invention can be a quencher that quenches the fluorescent dye. By using the quencher as the intercalator, the formation of a double-stranded nucleic acid can be detected based of a quench of fluorescence.

In the method according to the present invention, no particular limitation is imposed on an interaction system. For example, the first nucleic acid strand can be immobilized at an end thereof on a surface of a region of interaction.

The present invention also provides a method for obtaining information on hybridization, which includes conducting at least a step of allowing the hybridization to proceed between a first nucleic acid strand, on which a fluorescent dye is labeled, and a second nucleic acid strand having a base sequence to that of the first nucleic acid strand, a step of adding an intercalator which can be bound or inserted between base pairs in the double-stranded nucleic acid to permit an energy transfer with the fluorescent dye, and a step of detecting information on fluorescence of the fluorescent dye and intercalator.

The present invention further provides a method for obtaining information on an amount of a nucleic acid strand having a base sequence complementary to that of a probe nucleic acid strand, which including detecting information on fluorescence of a probe consisted of a labeling fluorescent dye labeled on the probe nucleic acid strand and an intercalator bound or inserted between base pairs in a double-stranded nucleic acid to permit an energy transfer with the fluorescent dye.

A description is now made of a term in the present invention.

The term "fluorescence resonance energy transfer (FRET)" as used herein means a phenomenon that, when the distance between a fluorescent dye as a donor and another fluorescent dye as an acceptor is in the neighborhood of approximately 1 nm to 10 nm, excitation of the donor causes energy to transfer to the acceptor and the acceptor is then excited.

According to the method of the present invention, it is possible to reduce background signals and to obtain information on the formation of a double-stranded nucleic acid with high detection sensitivity by a simple procedure that requires neither the fluorescent labeling of plural kinds of nucleic acid strands nor a washing step. In addition, the information on the formation of the double-stranded nucleic acid can be obtained without imposing a limitation on an interaction system.

### Brief Description of Drawings

[FIG. 1]

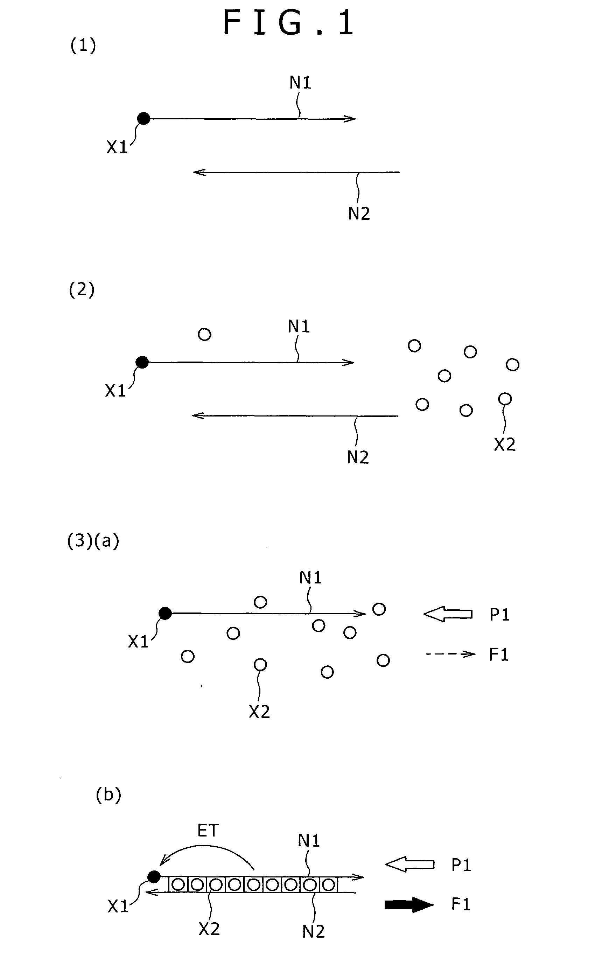

   FIG. 1 is a concept diagram of a method according to a first embodiment of the present invention for obtaining information on the formation of a double-stranded nucleic acid.
[FIG. 2]

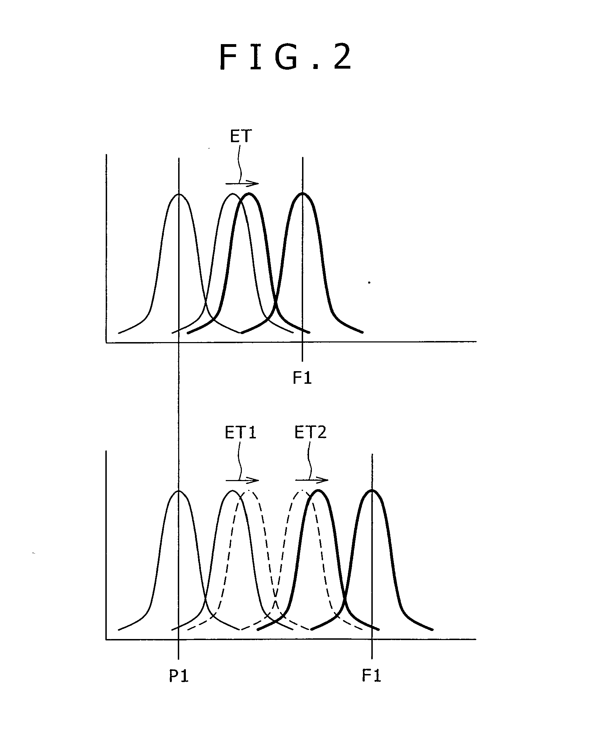

   FIG. 2 shows schematic diagrams of fluorescence spectra of a group of fluorescent dyes in the first embodiment of the present invention.
[FIG. 3]

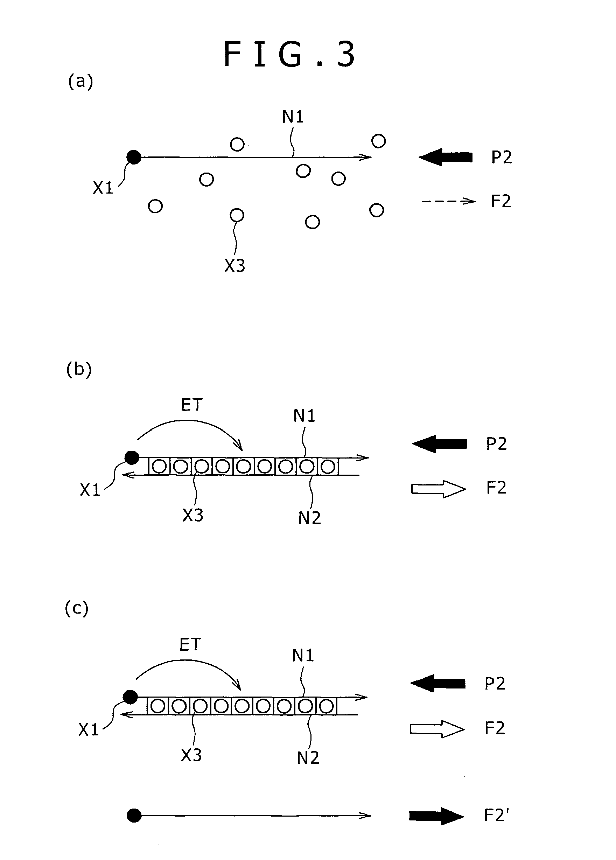

   FIG. 3 is a concept diagram of a method according to a second embodiment of the present invention for obtaining information on the formation of a double-stranded nucleic acid.
[FIG. 4]

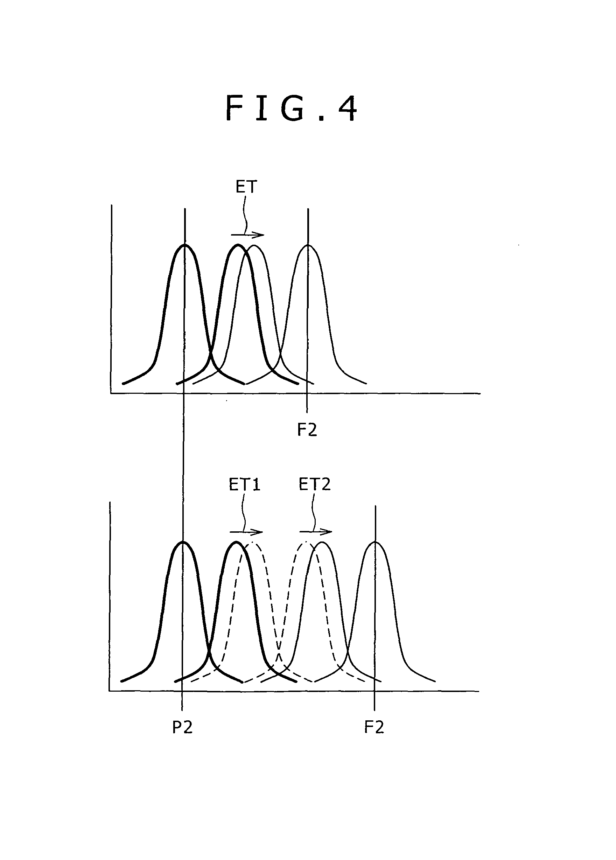

   FIG. 4 shows schematic diagrams of fluorescence spectra of a group of fluorescent dyes in the second embodiment of the present invention.
[FIG. 5]

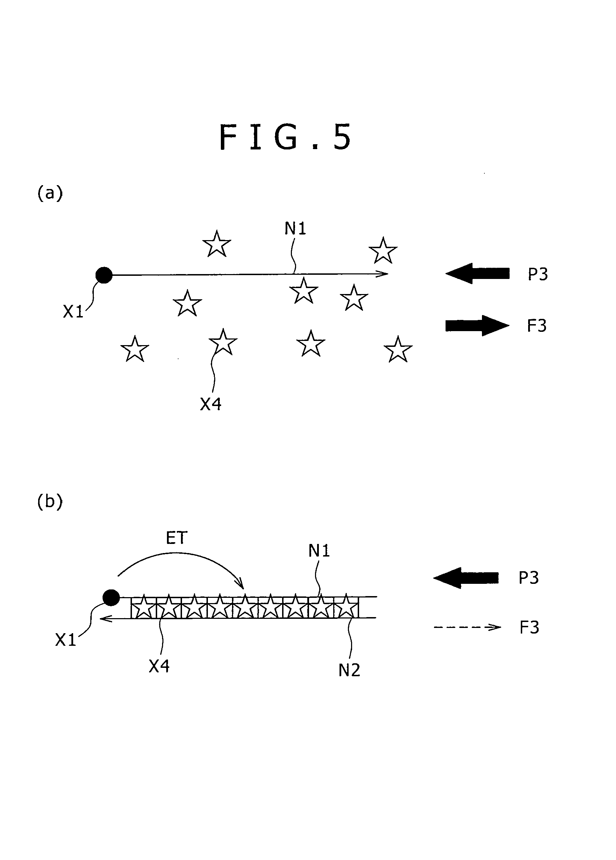

   FIG. 5 is a concept diagram of a method according to a third embodiment of the present invention for obtaining information on the formation of a double-stranded nucleic acid.
[FIG. 6]

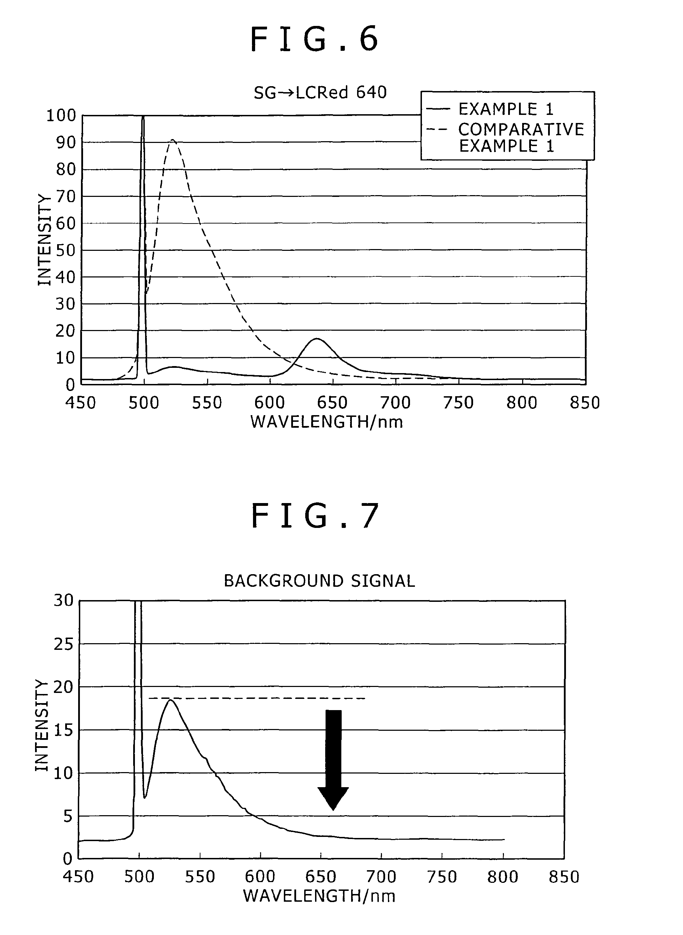

   FIG. 6 is fluorescence spectra relating to Example 1 of the present invention and Comparative Example 1.
[FIG. 7]
   FIG. 7 is a fluorescence spectrum relating to Example 2 of the present invention.
[FIG. 8]

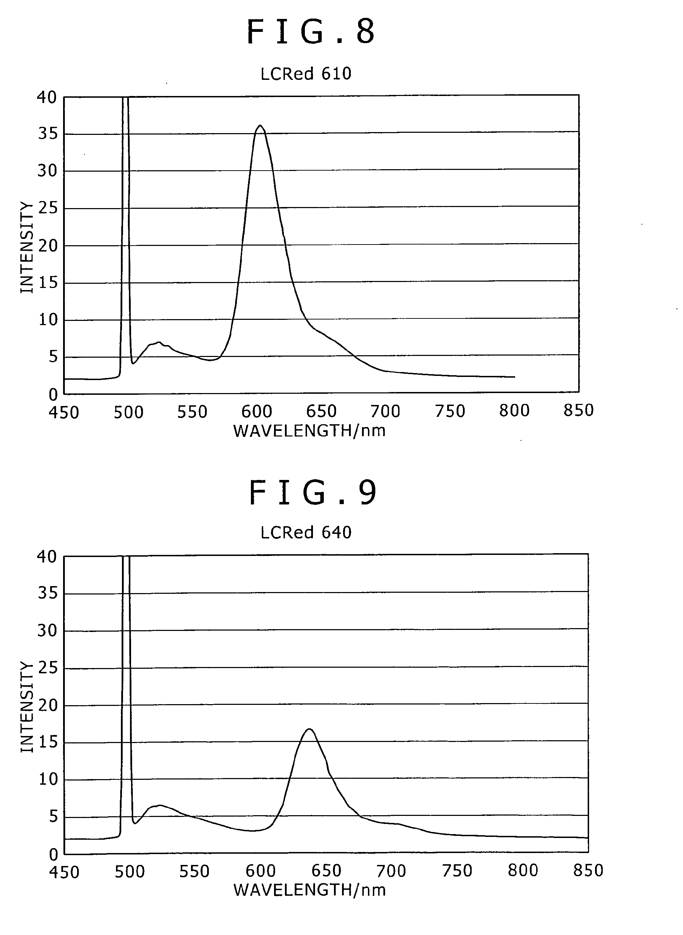

   FIG. 8 is a fluorescence spectrum relating to Example 3 of the present invention.
[FIG. 9]
   FIG. 9 is a fluorescence spectrum relating to Example 4 of the present invention.
[FIG. 10]

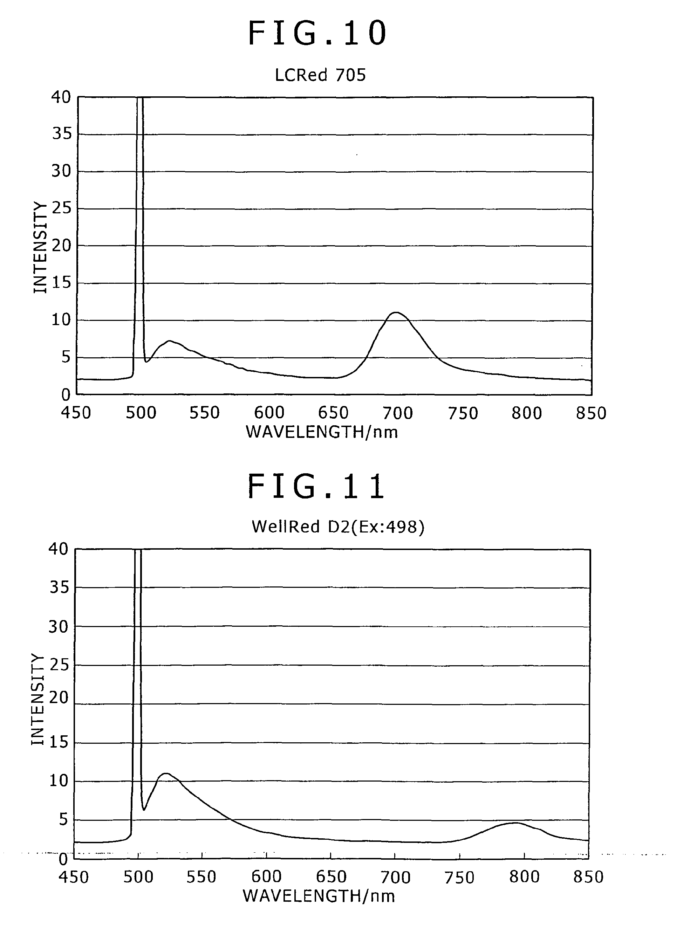

   FIG. 10 is a fluorescence spectrum relating to Example 5 of the present invention.
[FIG. 11]
   FIG. 11 is a fluorescence spectrum relating to Example 6 of the present invention.

### Best Modes for Carrying Out the Invention

With reference to the drawings, a description will hereinafter be made about preferred embodiments for carrying out the present invention. It is, however, to be noted that the embodiments to be described hereinafter are representative embodiments of the present invention by way of examples and that the scope of the present invention shall not be interpreted narrower by the embodiments.

FIG. 1 is a concept diagram of a method according to a first embodiment of the present invention, and illustrates a first nucleic acid strand N1, a second nucleic acid strand N2, a labeling fluorescent dye X1 and an intercalator X2 in the present invention.

The "probe" in the present invention is consisted of the labeling fluorescent dye X1 and intercalator X2 in this embodiment.

A general description will hereinafter be made about the first embodiment of the method according to the present invention.

In the method according to the present invention, hybridization is allowed to proceed between the first nucleic acid strand N1 labeled with the labeling fluorescent dye X1 and the second nucleic acid strand N2 having a base sequence complementary to that of the first nucleic acid strand N1 (see FIG. 1(1)).

Into the interaction system, the intercalator X2 is next added (see FIG. 1(2)). The intercalator X2 binds or inserts between base pairs in the double-stranded nucleic acid, and gives excitation energy to the labeling fluorescent dye X1 by a fluorescence resonance energy transfer ET.

In general, the intercalator X2 gives excitation energy to the labeling fluorescent dye X1 when the emission wavelength of the intercalator X2 and the excitation wavelength range for the labeling fluorescent dye X1 overlap.

Excitation light P1 of an excitation wavelength λExₓ₂ for the intercalator X2 as an excitation-target fluorescent dye is irradiated onto the interaction system, and fluorescence F1 is detected at a fluorescence detection wavelength λEmₓ₁ for the labeling fluorescent dye X1 as a detection-target fluorescent dye (see FIG. 1(3)).

As the emission wavelength range of the intercalator X2 is different from the fluorescence detection wavelength λEmₓ₁ for the labeling fluorescent dye X1, the intensity of fluorescence originated at λEmₓ₁ from the intercalator X2 is sufficiently low at this time. The excitation wavelength range for the labeling fluorescent dye X1 is also different from the excitation wavelength λExₓ₂ for the intercalator X2 so that, even when the excitation light P1 of λExₓ₂ is irradiated, the labeling fluorescent dye X1 is not substantially excited and the intensity of fluorescence emitted by direct excitation of the labeling fluorescent dye X1 is sufficiently low. Therefore, no substantial background signals are detected.

When the second nucleic acid strand N2 does not exist in the interaction system and no double-stranded nucleic acid is formed, the probability of occurrence of a fluorescence resonance energy transfer ET between the labeling fluorescent dye X1 and the intercalator X2, which constitute the probe, is sufficiently low because the labeling fluorescent dye X1 and intercalator X2 exist at random in the interaction system (see FIG. 1(3)(a)). Therefore, substantially no fluorescence is detected at λEmₓ₁ when the second nucleic acid strand N2 does not exist in the interaction system and the double-stranded nucleic acid is not formed.

When a double-stranded nucleic acid has been formed between the first nucleic acid strand N1 and the second nucleic acid strand N2, on the other hand, the intercalator X2 binds or inserts between base pairs in the double-stranded nucleic acid.

As a result, the intercalator X2 and the labeling fluorescent dye X1 labeled on the first nucleic acid strand N1, which constitute the probe, come close to each other to such a distance that a fluorescence resonance energy transfer ET occurs sufficiently (see FIG. 1(3)(b)).

When the light P1 of the excitation wavelength λExₓ₂ for the intercalator X2 is irradiated onto the interaction system in this state, a fluorescence resonance energy transfer ET occurs between the labeling fluorescent dye X1 and the intercalator X2 so that energy of the excited intercalator X2 transfers to the labeling fluorescent dye X1.

When the fluorescence resonance energy transfer ET occurs, the exited intercalator X2 is deactivated and the labeling fluorescent dye X1 fluoresces. Therefore, the fluorescence F1 originated from the labeling fluorescent dye X1 is detected at λEmₓ₁.

As the number of molecule pairs of the labeling fluorescent dye X1 and the intercalator X2 located close to each other becomes greater, in other words, the number of formed double-stranded nucleic acid molecules becomes greater, the probability of occurrence of the fluorescence resonance energy transfer ET increases, and as a result, the intensity of the fluorescence F1 increases.

It is, therefore, possible to obtain information on the formation of the double-stranded nucleic acid in the system by detecting the intensity of the fluorescence F1.

If a correlation between intensities of fluorescence F1 and amounts of the double-stranded nucleic acid in the system has been determined beforehand, an amount of the double-stranded nucleic acid formed in the system can be determined from an intensity of fluorescence F1. Using the first nucleic acid strand N1 as a probe, the method of the present invention thus makes it possible to obtain information on the amount of the second nucleic acid strand which takes part in the formation of the double-stranded nucleic acid.

A description will hereinafter be made about details of this embodiment.

No particular limitation is imposed on the kind of the first nucleic acid strand N1, and DNA, RNA, oligonucleotide or the like can be used.

The first nucleic acid strand N1 may be placed at any region of interaction. When employed in real-time PCR or the like, for example, it can be allowed to exist in a solution. When employed in a DNA-microarray or the like, on the other hand, it can be immobilized at an end thereof on a surface of a region of interaction.

No particular limitation is imposed on the kind of the labeling fluorescent dye X1 adapted to label the first nucleic acid strand N1 insofar as it receives excitation energy from the intercalator X2 by a fluorescence resonance energy transfer ET and emits. For example, "LightCycler^{™}Red 610," "LightCyclerRed 640," "WellRed D2," "Cy5" or the like can be used.

According to the present invention, it is sufficient to fluorescently label only the first nucleic acid strand N1. It is, therefore, possible to obtain information on the formation of a double-stranded nucleic acid with extreme ease compared with the method that requires labeling to plural kinds of nucleic acid strands or the method that requires plural kinds of labeling to one probe.

No particular limitation is imposed on the kind or the like of the second nucleic acid strand N2 insofar as it has a base sequence complementary to that of the first nucleic acid strand N1. Similar to the first nucleic acid strand N1, the second nucleic acid strand N2 can be DNA, RNA, oligonucleotide or the like.

Described specifically, the second nucleic acid strand N2 can be a target nucleic acid strand to be hybridized with the first nucleic acid strand N1, for example, in a DNA microarray or the like, or can be an amplification product to be produced by PCR, for example in real-time PCR or the like.

No particular limitation is imposed on the kind of the intercalator X2 insofar as the emission wavelength range of the intercalator X2 overlaps with the excitation wavelength range for the labeling fluorescent dye X1. The greater the overlap between the emission wavelength range of the intercalator X2 and the excitation wavelength range for the labeling fluorescent dye X1, the higher the probability of occurrence of the fluorescence resonance energy transfer ET.

As a specific example of the intercalator X2, an intercalator can be chosen, for example, from "SYBR^{™} GreenI," "YOYO-1," "LCGreenI^{™}" or the like as desired depending on the kind of the labeling fluorescent dye X1.

In the present invention, no particular limitation is imposed on the number of fluorescent dye(s) that constitute the probe. Although a single kind of fluorescent dye is used as the intercalator X2 in this embodiment, the intercalator X2 is only required to have at least an excitation-target fluorescent dye that gives excitation energy, which has been obtained by absorption of light, to another fluorescent dye, and can contain two or more kinds of fluorescent dyes.

Described specifically, it is possible to contain, for example, a fluorescent dye that receives excitation energy from an excitation-target fluorescent dye (ET1) and then gives the thus-received excitation energy to the labeling fluorescent dye X1 (ET2). Compared with the use of only an excitation-target fluorescent dye and a detection-target fluorescent dye (see the upper diagram in FIG. 2), the inclusion of such a fluorescent dye can broaden the wavelength difference between the wavelength of excitation light and the detection wavelength for fluorescence, and therefore, can reduce background signals (see the lower diagram in FIG. 2).

No particular limitation is imposed on a detection means for the fluorescence F1. For example, a fluorescence microscope, fluorometer, microarray scanner, real-time PCR system or the like can be used.

FIG. 3 is a concept diagram of a method according to a second embodiment of the present invention. A description about similar elements as in the first embodiment is omitted, and a description will be made about different elements. It is to be noted that symbol X3 in the diagram indicates an intercalator in this embodiment and that the "probe" in the present invention is consist of the labeling fluorescent dye X1 and the intercalator X3 in this embodiment.

In this embodiment, the intercalator X3 to be added into an interaction system binds or inserts between base pairs in a double-stranded nucleic acid, and receives excitation energy from the labeling fluorescent dye X1 by a fluorescence resonance energy transfer ET.

In general, the intercalator X3 receives the excitation energy from the labeling fluorescent dye X1 when the excitation wavelength range for the intercalator X3 and the emission wavelength range of the labeling fluorescent dye X1 overlap.

In this embodiment, excitation light P2 of a excitation wavelength λExₓ₁ for the labeling fluorescent dye X1 as an excitation-target fluorescent dye is irradiated onto the interaction system, and fluorescence F2 is detected at a fluorescence detection wavelength λEmₓ₃ for the intercalator X3 as a detection-target fluorescent dye (see FIG. 3).

As the emission wavelength range of the labeling fluorescent dye X1 is different from the fluorescence detection wavelength λEmₓ₃ for the intercalator X3, the intensity of fluorescence originated at λEmₓ₃ from the labeling fluorescent dye X1 is sufficiently low at this time. The excitation wavelength range for the intercalator X3 is also different from the excitation wavelength λExₓ₁ for the labeling fluorescent dye X1 so that, even when the excitation light P2 of λExₓ₁ is irradiated, the intercalator X3 is not substantially excited and the intensity of fluorescence emitted by direct excitation of the intercalator X3 is sufficiently low. Therefore, no substantial background signals are detected.

When the second nucleic acid strand N2 does not exist in the interaction system and no double-stranded nucleic acid is formed, the probability of occurrence of a fluorescence resonance energy transfer ET between the labeling fluorescent dye X1 and intercalator X3, which constitute the probe, is sufficiently low because the labeling fluorescent dye X1 and intercalator X3 exist at random in the interaction system (see FIG. 3(a)). Therefore, substantially no fluorescence is detected at λEmₓ₃ when the second nucleic acid strand N2 does not exist in the interaction system and the double-stranded nucleic acid is not formed.

When a double-stranded nucleic acid has been formed between the first nucleic acid strand N1 and the second nucleic acid strand N2, on the other hand, the intercalator X3 binds or inserts between base pairs in the double-stranded nucleic acid.

As a result, the intercalator X3 and the labeling fluorescent dye X1 labeled on the first nucleic acid strand N1, which constitute the probe, come close to each other to such a distance that a fluorescence resonance energy transfer ET occurs sufficiently (see FIG. 3(b)).

When the light P2 of the excitation wavelength λExₓ₁ for the labeling fluorescent dye X1 is irradiated onto the interaction system in this state, a fluorescence resonance energy transfer ET occurs between the labeling fluorescent dye X1 and the intercalator X3 so that energy of the excited labeling fluorescent dye X1 transfers to the intercalator X3.

When the fluorescence resonance energy transfer ET occurs, the exited labeling fluorescent dye X1 is deactivated and the intercalator X3 fluoresces. Therefore, the fluorescence F2 originated from the intercalator X3 is detected at λEmₓ₃.

As the number of molecule pairs of the labeling fluorescent dye X1 and the intercalator X3 located close to each other becomes greater, in other words, the number of formed double-stranded nucleic acid molecules becomes greater, the probability of occurrence of the fluorescence resonance energy transfer ET increases, and as a result, the intensity of the fluorescence F2 increases.

It is, therefore, possible to obtain information on the formation of the double-stranded nucleic acid in the system by detecting the intensity of the fluorescence F2.

By detecting fluorescence F2' at the fluorescence detection wavelength λEmₓ₁ for the labeling fluorescent dye X1 concurrently with the fluorescence F2 at λEmₓ₃, it is possible to concurrently obtain information on the labeling fluorescent dye X1 which does not cause a fluorescence resonance energy transfer ET with the intercalator X3, in other words, the first nucleic acid strand N1 which does not form a double-stranded nucleic acid with the second nucleic acid strand N2 (see FIG. 3(c)).

From the ratio in fluorescence intensity of the fluorescence F2' at λEmₓ₁ to the fluorescence F2 at λEmₓ₃, the percentage of the double-stranded nucleic acid formed between the first nucleic acid strand N1 and the second nucleic acid strand N2 can be determined.

A description will hereinafter be made about details of this embodiment.

No particular limitation is imposed on the kind of the labeling fluorescent dye X1 adapted to label the first nucleic acid strand N1 insofar as it gives excitation energy, which has been obtained by absorbing the excitation light P2, to the intercalator X3 by a fluorescence resonance energy transfer ET. For example, FITC or the like can be used.

No particular limitation is imposed on the kind of the intercalator X3 insofar as the emission wavelength range of the intercalator X3 overlaps with the emission wavelength range of the labeling fluorescent dye X1. The greater the overlap between the excitation wavelength range for the intercalator X3 and the emission wavelength range of the labeling fluorescent dye X1, the higher the probability of occurrence of the fluorescence resonance energy transfer ET.

As the intercalator X3, an intercalator can be chosen, for example, from "TOTO-3," "TO-PRO-3," "YOYO-3," "YO-PRO-3" or the like as desired depending on the kind of the labeling fluorescent dye X1.

In the present invention, no particular limitation is imposed on the number of fluorescent dye(s) that constitute the probe. Although a single kind of fluorescent dye is used as the intercalator X3 in this embodiment, the intercalator X3 is only required to have at least a detection-target fluorescent dye that received excitation energy from another fluorescent dye and fluoresces, and can contain two or more kinds of fluorescent dyes.

Described specifically, it is possible to contain, for example, a fluorescent dye that receives excitation energy from the labeling fluorescent dye X1 as an excitation-target fluorescent dye by a fluorescence resonance energy transfer ET (ET1) and then gives the energy to a detection-target fluorescent dye (ET2). Compared with the use of only an excitation-target fluorescent dye and a detection-target fluorescent dye (see the upper diagram in FIG. 4), the inclusion of such a fluorescent dye can broaden the wavelength difference between the wavelength of excitation light and the detection wavelength for fluorescence, and therefore, can reduce background signals (see the lower diagram in FIG. 4).

FIG. 5 diagrammatically illustrates a third embodiment of the method according to the present invention. A description about similar elements as in the first embodiment is omitted, and a description will be made in detail about different elements. It is to be noted that symbol X4 in the diagram indicates an intercalator in this embodiment and that the "probe" in the present invention is consist of the labeling fluorescent dye X1 and the intercalator X4 in this embodiment.

In this embodiment, the intercalator X4 to be added into an interaction system binds or inserts between base pairs in a double-stranded nucleic acid, and receives excitation energy from the labeling fluorescent dye X1 by a fluorescence resonance energy transfer ET to quench the labeling fluorescent dye X1.

It is to be noted that no particular limitation is imposed on the kind of the labeling fluorescent dye X1 adapted to label the first nucleic acid strand N1 insofar as it gives excitation energy, which has been obtained as a result of absorption of the excitation light P3, to the intercalator X4 by a fluorescence resonance energy transfer ET and is quenched, and also, that no particular limitation is imposed on the kind of the intercalator X4 insofar as it receives the excitation energy for the labeling fluorescent dye X1 by a fluorescence resonance energy transfer ET to quenches the labeling fluorescent dye X1.

In this embodiment, excitation light P3 of the excitation wavelength λExₓ₁ for the labeling fluorescent dye X1 as an excitation-target fluorescent dye is irradiated onto the interaction system, and fluorescence F3 is detected at the fluorescence detection wavelength λEmₓ₁ for the labeling fluorescent dye X1 (see FIG. 5).

When the second nucleic acid strand N2 does not exist in the interaction system and the double-stranded nucleic acid is not formed, the probability of occurrence of a fluorescence resonance energy transfer ET between the labeling fluorescent dye X1 and intercalator X4 is sufficiently low because the labeling fluorescent dye X1 and intercalator X4 exist at random in the interaction system (see FIG. 5(a)). Therefore, the labeling fluorescent dye X1 is not quenched and fluorescence F3 is detected at λEmₓ₁, when the second nucleic acid strand N2 does not exist in the interaction system and the double-stranded nucleic acid is not formed.

When a double-stranded nucleic acid has been formed between the first nucleic acid strand N1 and the second nucleic acid strand N2, on the other hand, the intercalator X4 binds or inserts between base pairs in the double-stranded nucleic acid.

As a result, the intercalator X4 bound or inserted between the base pairs in the double-bonded nucleic acid and the labeling fluorescent dye X1 labeled on the first nucleic acid strand N1 come close to each other to such a distance that a fluorescence resonance energy transfer ET occurs sufficiently (see FIG. 5(b)).

When the light P3 of the excitation wavelength λExₓ₁ for the labeling fluorescent dye X1 is irradiated onto the interaction system in this state, a fluorescence resonance energy transfer ET occurs between the labeling fluorescent dye X1 and the intercalator X4 so that energy of the excited labeling fluorescent dye X1 transfers to the intercalator X4.

When the fluorescence resonance energy transfer ET occurs, the exited labeling fluorescent dye X1 is deactivated and is quenched. Therefore, the fluorescence F3 is not detected at λEmₓ₁.

As the number of molecule pairs of the labeling fluorescent dye X1 and the intercalator X4 located close to each other becomes greater, in other words, the number of formed double-stranded nucleic acid molecules becomes greater, the probability of occurrence of the fluorescence resonance energy transfer ET increases, and therefore, the intensity of the fluorescence F3 decreases.

It is, therefore, possible to obtain information on the formation of the double-stranded nucleic acid in the system by detecting the intensity of the fluorescence F3.

If a correlation between intensities of fluorescence F3 and amounts of the first nucleic acid strand N1, which does not form the double-stranded nucleic acid, has been determined beforehand, an amount of the first nucleic acid strand N1 that does not form the double-stranded nucleic acid in the system can be determined from an intensity of fluorescence F3.

### Examples

The present invention will hereinafter be described in detail based on examples, although the present invention shall not be limited to the following examples.

Conducted first was to confirm that information on the formation of a double-stranded nucleic acid can be obtained by the method according to the present invention.

### <Example 1>

To a solution containing a nucleic acid strand N1 of 18-mer base sequence fluorescently labeled with a fluorescent dye, "LightCyclerRed 640" (product of Roche Diagnostics K.K.," a solution containing a nucleic acid strand N2 having a base sequence complementary to that of the nucleic acid strand N1 and "SYBR^{™} Green I" were added, followed by mixing. After the resulting mixture was heated at 95°C for 1 minute and then incubated at 20°C for 5 minutes, a fluorescence spectrum was measured with a fluorescence spectrophotometer by setting an excitation wavelength at 498 nm which is the excitation wavelength for "SYBR^{™} Green I." During the measurement, the interaction solution was controlled to remain at 20°C.

Experimental conditions are shown in Table 2.

**[Table 1]**

| | |
|---|---|
| Solution | 100 mM NaCl |
| composition | 10 mM Na₃PO₄ |
| | 0.1 mM EDTA |
| DNA | N1: 1 µM |
| concentration | N2: 1 µM |
| DNA sequence | N1: CGAAGTGCAGGGCAGATC |
| | N2: GATCTGCCCTGCACTTCG |
| Intercalator | "SYBR Green I" |
| | (CAMBREX Bio Science Rockland Inc., |
| | Cat. #50513) |
| | 10,000-fold dilution |
| Fluorometer | "HITACHI F-4500 MODEL" fluorescence spectrophotometer |
| | Measurement mode: wavelength scanning |
| | Scan mode: fluorescence spectrum |
| | Excitation wavelength: 498.0 nm |
| | Fluorescent emission start wavelength: |
| | 450.0 nm |
| | Fluorescent emission end wavelength: |
| | 800.0 nm |
| | Scan speed: 240 nm/min |
| | Excitation slit: 2.5 nm |
| | Fluorescence slit: 2.5 nm |
| | Photomultiplier voltage: 700 V |
| | Response: 0.5 s |

### <Comparative Example 1>

Using a fluorescently-unlabeled nucleic acid strand N1, a similar experiment as in Example 1 was conducted.

Fluorescence spectra of Example 1 and Comparative Example 1 are shown in FIG. 6.

Fluorescence (520 nm) originated from "SYBR^{™} Green I," which is confirmed in the fluorescence spectrum of Comparative Example 1, was not detected practically in the fluorescence spectrum of Example 1. Instead, fluorescence (640 nm) of "LightCyclerRed 640" labeled on the nucleic acid strand N1 appeared.

In Example 1, "SYBR^{™} Green I" bound or inserted as an intercalator between the base pairs in the double-stranded nucleic acid formed between the nucleic acid strands N1 and N2, and therefore, came sufficiently close to "LightCyclerRed 640" labeled on the nucleic acid strand N1. It is presumed that as a result of excitation of "SYBR^{™} Green I" as the intercalator in that state, FRET occurred between the intercalator and "LightCyclerRed 640" labeled on the nucleic acid strand N1 and fluorescence of "LightCyclerRed 640" was detected.

From the above-described results, it has been confirmed that information on a double-stranded nucleic acid can be obtained by the method according to the present invention.

Conducted next was to confirm that a reduction of background signals is feasible by the method according to the present invention.

### <Example 2>

To a solution containing a fluorescently-unlabeled nucleic acid strand N1, a solution containing a nucleic acid strand N3 (base sequence: TGCCCACTATTAAGGAAGG), which forms no double-stranded nucleic acid with the nucleic acid strand N1, and "SYBR^{™} Green I" were added, and a fluorescence spectrum was measured. Experimental conditions such as the solution composition were set similarly as in Example 1.

A fluorescence spectrum of Example 2 is shown in FIG. 7.

It is appreciated that despite the formation of no double-stranded nucleic acid, fluorescence was recognized at the emission wavelength (520 nm) of "SYBR^{™} Green I" and background signals wee observed. As the background signals reached the maximum at 520 nm and were then reduced progressively as the wavelength shifted, the selection of a detection-target fluorescent dye in the method according to the present invention is believed to reduce background signals.

A comparison was then made in fluorescence intensity by changing the kind of the fluorescent dye adapted to label the nucleic acid strand N1. Hybridization and the measurement of fluorescence spectra were conducted similarly as in Example 1. Conditions for the respective examples are shown in Table 1.

**[Table 2]**

| | Excitation-target fluorescent dye | | Detection-target fluorescent dye | |
|---|---|---|---|---|
| | Fluorescent dye | Excitation wavelength (nm) | Fluorescent dye | Emission wavelength (nm) |
| Example 3 | "SYBR Green I" | 494 | "LCRed 610" | 610 |
| Example 4 | | | "LCRed 640" | 740 |
| Example 5 | | | "LCRed 705" | 705 |
| Example 6 | | | "WellRed D2" | 770 |

Fluorescence spectra of Examples 3 through 6 are shown in FIGS. 8 through 11, respectively.

Comparing the fluorescent spectra of FIGS. 8 through 11, the emission intensity of the detection-target fluorescent dye decreases in the order of Example 3, Example 4, Example 5 and Example 6, with Example 3 being the highest. Comparing the detection-target fluorescent dyes employed in the respective examples, it is appreciated that the fluorescence intensity increases as the excitation wavelength for the employed detection-target fluorescent dye becomes closer to the emission wavelength of "SYBR^{™} Green I."

From the above-described results, it is presumed that, as the overlap between the emission wavelength of an excitation-target fluorescent dye and the excitation wavelength for a detection-target fluorescent dye becomes greater, the probability of occurrence of FRET increases, and as a result, the fluorescence intensity of the detection-target fluorescent dye increases.

### Industrial Applicability

Use of the method according to the present invention makes it possible to obtain information on the formation of a double-stranded nucleic acid and information on the amount of a nucleic acid in various systems such as Southern blotting, Northern blotting, DNA microarray, real-time PCR, ICAN, LAMP, TRC and the like. In particular, the method according to the present invention is a simple method that requires neither fluorescent labeling to plural kinds of nucleic acid strands nor a washing step, reduces background signals, and can obtain information on the formation of a double-stranded nucleic acid with high detection sensitivity, and therefore, is industrially useful.

## Claims

1. A method for obtaining information on formation of a double-stranded nucleic acid between a first nucleic acid strand and a second nucleic acid strand having a base sequence complementary to that of the first nucleic acid strand, which comprises detecting information on fluorescence of a probe consisted of a labeling fluorescent dye labeled on the first nucleic acid strand and an intercalator bound or inserted between base pairs in the double-stranded nucleic acid to permit an energy transfer with the fluorescent dye.

2. The method according to claim 1, wherein the probe is comprised of a group of plural fluorescent dyes including at least an excitation-target fluorescent dye capable of giving excitation energy, which has been obtained by absorption of light, to another fluorescent dye and a detection-target fluorescent dye capable of fluorescing by receiving the excitation energy from the another fluorescent dye.

3. The method according to claim 1, wherein the intercalator is a quencher that quenches the labeling fluorescent dye.

4. The method according to claim 1, wherein the first nucleic acid strand is immobilized at an end thereof on a surface of a region of interaction.

5. A method for obtaining information on hybridization, which comprises conducting at least:
a step of allowing the hybridization to proceed between a first nucleic acid strand, on which a fluorescent dye is labeled, and a second nucleic acid strand having a base sequence complementary to that of the first nucleic acid strand,
a step of adding an intercalator which can be bound or inserted between base pairs in the double-stranded nucleic acid to permit an energy transfer with the fluorescent dye, and
a step of detecting information on fluorescence of the fluorescent dye and intercalator.

6. A method for obtaining information on an amount of a nucleic acid strand having a base sequence complementary to that of a probe nucleic acid strand, which comprising detecting information on fluorescence of a probe consisted of a labeling fluorescent dye labeled on the probe nucleic acid strand and an intercalator bound or inserted between base pairs in a double-stranded nucleic acid to permit an energy transfer with the fluorescent dye.
